Europäisches Patentamt

European Patent Office

Office européen des brevets

(11) Numéro de publication: **0 392 951**
**A2**

(12) **DEMANDE DE BREVET EUROPEEN**

(21) Numéro de dépôt: 90430008.4

(51) Int. Cl.5: **A61C 1/00, A61C 5/00**

(22) Date de dépôt: **10.04.90**

(30) Priorité: **10.04.89 US 335245**

(43) Date de publication de la demande:
**17.10.90 Bulletin 90/42**

(84) Etats contractants désignés:
**AT BE CH DE DK ES FR GB IT LI NL SE**

(71) Demandeur: **Levy, Guy**
**49, rue Croix de Regnier**

**F-13004 Marseille(FR)**

(72) Inventeur: **Levy, Guy**
**49, rue Croix de Regnier**
**F-13004 Marseille(FR)**

(74) Mandataire: **Marek, Pierre**
**28 & 32 rue de la Loge**
**F-13002 Marseille(FR)**

(54) **Appareil et procédé utilisant l'effet laser, pour la vaporisation ou la fusion de matériaux et de tissus divers.**

(57) Appareil utilisant l'effet laser pour la vaporisation ou la fusion de matériaux divers dans un environnement fragile ou délicat, notamment : - pour le traitement de la carie dentaire ; - ou pour la découpe et la sculpture de matériaux divers utilisés en art dentaire ou non tel que métaux, ivoire, émail, os ; - ou pour la fusion, sur la dent, d'un composé minéral à base d'hydroxyapatite et de céramique ; - ou pour l'élargissement des canaux dentaires à l'aide d'une fibre optique conduisant l'énergie laser ; caractérisé en ce qu'il comprend un laser spécifique de type ND YAG présentant, en combinaison, les caractéristiques ci-après :
- une longueur d'onde de l'ordre de 1,060 μm ;
- une capacité d'émission d'une énergie pulsée comprise entre 0 et 400 millijoules ;
- des durées de pulsations ou impulsions comprises entre 0,3 et 1,2 millisecondes ;
- un taux de répétition minimal de 50 Hz ;
ledit appareil comportant également une pièce à main agencée pour recevoir une fibre optique souple (8) dont une partie s'étend hors de ladite tête pour l'application du rayonnement laser, et pour permettre de projeter un mélange air/eau sur le site d'intervention.

Fig.1

La présente invention concerne un appareil et un procédé utilisant l'effet laser, pour la vaporisation ou la fusion de matériaux et de tissus divers tels que métaux, bois , peaux, émail, ivoire, os, etc, dans un environnement délicat ou fragile.

Selon des exemples d'applications avantageuses, mais non limitatives, l'installation et le procédé selon l'invention peuvent être mis en oeuvre pour l'exécution de certaines interventions de l'art dentaires, telles que :
- la découpe et la sculpture des matières dentinaires et osseuses ;
- la coupe des objets métalliques à l'intérieur de la dent ou de la bouche ;
- la coupe des tissus vascularisés ;
- l'induction d'un plasma ;
- la fusion, sur la dent, d'un composé minéral à base d'Hydroxyapatite et de céramique ;
- l'élargissement des canaux dentaires.

Dans le traitement de diverses affections dentaires et autres maladies, il est fréquemment nécessaire d'enlever la matière osseuse de la racine, la dentine ou le cément et il est souhaitable de le faire sans entrainer d'effets secondaires nuisibles pour le patient.

En exécutant des procédures médicales à l'intérieur de la cavité buccale, le praticien est souvent confronté à la présence de corps métalliques introduits lors de traitements dentaires antérieurs, ces corps étant constitués par des obturations et broches métalliques ou des pivots en chrome utilisés pour maintenir les prothèses en place, et il convient de couper ces éléments, toujours sans entraîner d'effets secondaires nuisibles dans les tissus environnants.

De même, les praticiens rencontrent fréquemment des kystes et granulomes, qui se développent dans la gencive près de l'apex d'une dent, et il est nécessaire de détruire ces excroissances ou, au moins, de les réduire de façon substantielle.

En outre, s'il existe désormais bon nombre de matériaux d'obturation dentaire, on constate le besoin constant d'une matière capable d'obturer non seulement les cavités dentaires, mais également celles qui existent, ou ont été créées, dans la matière osseuse, et qui présenterait une dureté comparable à celle de la matière qu'elle remplace, cette matière devant être, en outre, capable de s'accrocher solidement sur la paroi de la cavité ou de l'orifice.

Les procédés et appareils connus utilisant l'effet laser ne permettent pas de réaliser les opérations susmentionnées de manière satisfaisante.

Le procédé décrit dans le document US-A-4.521.194 prévoit l'utilisation d'un laser de type ND YAG pour l'élimination de la carie.

La méthode décrite dans ce document ne permet pas la coupe de l'émail, ou de la dentine, ou de l'os, ni des éléments métalliques qui peuvent être contenus dans la dent ; elle ne permet pas, non plus, le contrôle de l'énergie thermique.

Le dispositif et la méthode décrits dans le document US-A-4.273.535 donnent lieu à des observations identiques.

Le document US-A-4.826.431 concerne une pièce à main comprenant un générateur de rayon laser à $CO_2$, transmis au moyen d'une fibre optique, et un système de refroidissement pour ce générateur ; cette pièce à main peut être complétée par un système de projection d'un fluide de refroidissement tel que : air et/ou eau, associé ou non à l'émission, pour refroidir la fibre et pour nettoyer la zone à irradier. Toutefois, la fonction de la pièce à main décrite dans ce document, n'est pas de refroidir la dent, ni de travailler à des énergies relativement élevées d'une façon efficace, une telle fonction ne pouvant être assurée en utilisant le système de refroidissement avec l'émission $CO_2$ car l'onde à 10,6 $\mu$m est absorbée totalement par l'eau et n'aurait donc aucune utilité.

La présente invention a pour objet un appareil et un procédé permettant la mise en oeuvre effective du rayonnement laser en vue d'améliorer le mode opératoire des procédures précédemment décrites.

Un objet plus spécifique de l'invention consiste à utiliser le rayonnement laser de façon sélective pour couper l'os, la dentine, le cément et la racine dentaire, ainsi que les corps métalliques qui peuvent se trouver dans la dent ou dans la bouche, sans exposer le patient à des effets secondaires nuisibles, notamment par brûlures du tissu attenant au site d'intervention.

Un autre objet de l'invention consiste à fournir un matériau d'obturation innovant pour remplir les cavités ou les orifices dans les dents et dans les os, et d'utiliser le rayonnement laser pour favoriser le durcissement d'un tel matériau d'obturation.

Un nouvel objet de l'invention consiste à apporter un traitement amélioré des kystes et granulomes des os et des gencives.

Selon l'un des aspects de l'invention, les objectifs ci-dessus sont réalisés grâce à une méthode de remplissage d'une ouverture dans le matériau tel que, par exemple, la matière osseuse ou dentaire, qui consiste à : - former une pâte composée d'un mélange de liquide et de poudre contenant de l'hydroxyapatite ; -remplir l'orifice à l'aide de la pâte, et ; - irradier la pâte qui remplit l'orifice par un rayonnement laser afin d'accrocher l'hydroxyapatite à la matière qui entoure l'orifice.

Suivant un autre aspect de l'invention, ces objectifs se réalisent grâce à un appareil et à une méthode de traitement d'un kyste ou d'un granulome dans la gencive, situé à l'apex du canal dentaire, qui consiste à : - ouvrir le canal près de l'apex, insérer dans le canal une fibre optique de telle sorte que son extrémité active se trouve sur l'apex ; et envoyer une succession d'impulsions irradiantes à travers la fibre de telle sorte que le rayonnement sorte par l'extrémité, attaque et ouvre le foramen et, ensuite, attaque et au moins réduise le kyste ou le granulome.

Selon un autre aspect de l'invention, les objectifs ci-dessus peuvent être réalisés grâce à un appareil et à une méthode permettant de couper la matière osseuse, la dentine, le cément et la racine dentaire, et qui consiste à : produire un rayonnement laser de longueur d'ondes adéquate pour couper une telle matière ; produire des impulsions successives du rayonnement laser véhiculant une quantité d'énergie, pendant une durée et sous une fréquence choisies de manière à pouvoir couper la matière sans provoquer d'effets secondaires nuisibles ; concentrer les impulsions sur un point suffisamment petit pour entraîner la découpe de la matière et, en même temps, concentrer et diriger un fluide de refroidissement sur le site.

Suivant un autre aspect de l'invention, les objectifs ci-dessus se réalisent grâce à un appareil et à une méthode de découpe des corps métalliques rencontrés dans les dents ou la bouche d'un patient, qui consiste à : produire un rayonnement laser de longueur d'ondes adéquate pour couper le métal ; produire des impulsions successives de rayonnement véhiculant une quantité d'énergie, pendant une durée et à une fréquence choisies de manière à couper le métal sans produire d'effets secondaires nuisibles ; concentrer les rayonnements sur le métal sur un point suffisamment petit et, simultanément, concentrer et diriger un fluide de refroidissement sur le site d'intervention.

L'appareil selon l'invention est notamment remarquable en ce qu'il comprend un moyen de production d'un rayonnement laser sur une longueur d'ondes permettant une plus forte absorption par l'hydroxyapatite que par l'eau ; un moyen associé audit moyen de production tel que ce dernier produise une succession d'impulsions du rayonnement produit, qui présentent une quantité d'énergie, une durée d'impulsion et une fréquence d'impulsions choisies de manière à vaporiser, à couper ou à fondre le matériau à traiter, sans entrainer d'effets secondaires nuisibles dans la matière environnante ; et un moyen permettant de concentrer le rayonnement sur le matériau à traiter en un point suffisamment petit pour provoquer la vaporisation ou la découpe, ou la fusion de ce dernier.

Plus précisément, l'appareil selon l'invention est constitué par un laser spécifique de type ND YAG présentant, en combinaison, les caractéristiques ci-après :
- une longueur d'onde de 1,060 microns ($\mu$m) ;
- une capacité d'émission d'une énergie pulsée comprise entre 0 et 400 millijoules ;
- un taux de répétition minimal de 50 Hz ;
- des durées de pulsation comprises entre 0.3 et 1.2 millisecondes.

Selon une autre disposition caractéristique, l'appareil selon l'invention comprend une pièce à main comportant une fibre optique pour le transport et l'application du rayon laser, cette pièce à main permettant, en outre, de délivrer un jet d'air et/ou d'eau sur le site d'intervention, en même temps que le rayon laser, pour refroidir la zone de coupe et éviter tout effet thermique néfaste à distance.

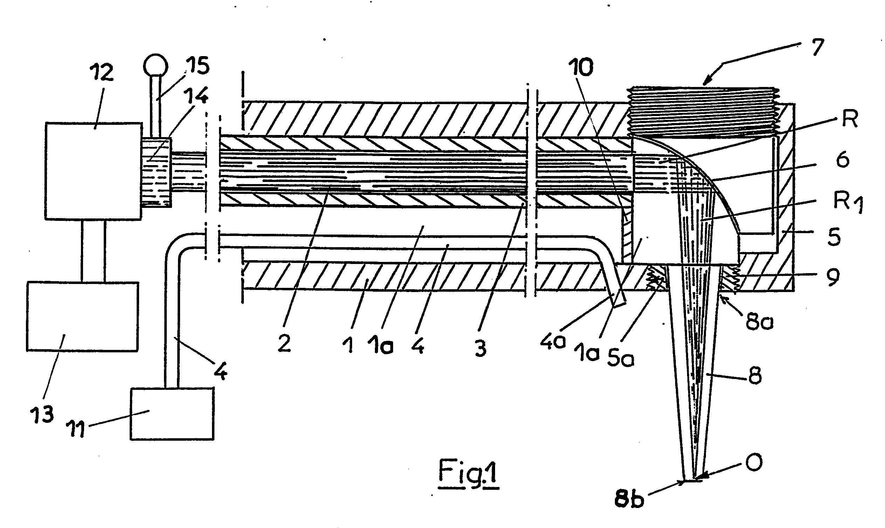

L'unique figure du dessin annexé illustre, par une vue à caractère schématique et en coupe longitudinale, une pièce à main permettant l'application d'une énergie laser focalisée sur une surface d'intervention, et la projection d'un mélange air/eau de refroidissement sur cette surface.

Selon un premier aspect de l'invention, on constitue un matériau d'obturation à l'aide d'un mélange comportant un composant liquide formé d'acide phosphorique et d'eau et d'un composant pulvérulent composé de céramique et d'hydroxyapatite, en mélangeant les ingrédients dans des proportions permettant de constituer une pâte d'une consistance suffisante pour pouvoir être travaillée et suffisamment plastique pour être appliquée dans une cavité d'une matière environnante, à l'aide d'une spatule et rester en place, et on applique ensuite le rayonnement laser présentant les caractéristiques décrites ci-après afin de solidifier et de durcir le mélange et de le faire accrocher sur ladite matière environnante.

Les proportions du mélange ne sont pas décisives ; toutefois, elles sont, de préférence, établies comme suit :

| Liquide : | Acide phosphorique | 40 % |
|---|---|---|
| | Eau | 60 % |
| Poudre : | Céramique | 80 % |
| | Hydroxyapatite | 20 % |

Si on augmente la teneur en hydroxyapatite, il faut davantage d'énergie pour durcir le mélange ; si la proportion d'hydroxyapatite diminue, la résistance de la liaison s'amoindrit.

Le composant de la céramique peut, par exemple, être constitué par la cordiérite, la silice, l'oxyde de silice, l'oxyde d'aluminium. Dans l'application du procédé à l'art dentaire, les composants pulvérulents ont la granulométrie des matériaux dentaires de remplissage employés habituellement en dentisterie.

On mélange les composants liquide et pulvérulent juste avant l'introduction dans la cavité à remplir.

La longueur d'onde du rayonnement appliqué au cours de ce traitement est de 1,06 μm, et il se compose d'impulsions d'une durée égale à 0,4 ms, de préférence, sous une fréquence de l'ordre de 50 Hz, et véhiculant une énergie de l'ordre de 20 mJ par impulsion. Toutefois, contrairement aux différentes opérations de coupe qui seront décrites en détail ci-après, le faisceau doit être ici défocalisé de manière à devenir au moins coextensif avec la surface exposée du matériau d'obturation. Cette disposition s'obtient facilement en faisant varier l'écartement entre la surface rayonnante à la sortie de la pièce à main et la surface à traiter, afin que le rayonnement recouvre au moins une partie de la surface exposée de la pâte qui obture l'orifice.

L'exposition du matériau de remplissage au rayonnement laser permet le développement d'une structure cristalline dans ce matériau et créé un accrochage solide entre l'hydroxyapatite et le matériau environnant de la surface à restaurer.

Le rayonnement est appliqué jusqu'à ce qu'apparaisse une structure cristalline, ce qui, en général, nécessite une exposition de l'ordre de 10 à 30 secondes.

Le matériau d'obturation et le rayonnement décrits ci-avant peuvent servir à remplir des brisures ou trous de la matière de la surface à restaurer.

Selon un autre aspect de l'invention, le rayonnement présentant les caractéristiques décrites ci-avant peut être utilisé pour traiter un kyste ou un granulome attenant à l'apex d'une dent. A cet effet, après avoir ouvert le canal jusqu'au foramen, on passe à l'intérieur du canal une fibre optique d'un diamètre de l'ordre de 200 microns par exemple, et on envoie, par son intermédiaire, un rayonnement présentant les caractéristiques décrites ci-dessous pour l'incision des tissus durs, afin d'inciser le foramen et d'éliminer le kyste ou le granulome.

Pour cette opération, il est préférable d'utiliser un rayonnement de longueur d'ondes égale à 1,06 μm, une durée d'impulsions de l'ordre de 0,4 ms, une fréquence d'impulsions de l'ordre de 50 hz et une quantité d'énergie de 50 à 100 mJ par impulsion.

Selon une autre disposition caractéristique de l'invention, il est possible de couper, sans brûlure, l'os, la racine, la dentine et le cément en appliquant pendant quelques secondes un rayonnement du type défini plus haut, tout en irrigant avec un mélange d'air et d'eau afin de maitriser l'effet de coupe thermique du faisceau laser. Dans ce cas, la longueur d'onde du rayonnement est de 1,06 μm, la durée d'impulsion est de l'ordre de 0,8 à 1,2 ms, la fréquence est de l'ordre de 50 Hz et l'énergie par impulsion est de 200 à 400 mJ. Cette opération peut être pratiquée sans former au préalable une zone sombre au point d'induction.

Toutefois, la formation d'une zone sombre au point d'induction augmente l'absorption d'énergie et permet d'effectuer la coupe plus rapidement. En outre, on peut créer un point sombre à l'aide d'un faisceau à faible énergie si l'on souhaite tracer ou délimiter au préalable la zone à découper.

De plus, un rayonnement de la forme indiquée ci-avant pour découper l'os peut également servir à découper les pièces métalliques dans la cavité buccale, tels que plombages, broches ou pivots en chrome de prothèses dentaires. Pour ce faire, on crée et dirige le rayonnement laser sur le matériau à couper, de la manière décrite plus haut.

Pour exécuter toutes les opérations qui viennent d'être décrites, la surface de sortie lumineuse de la pièce à main sera disposée de manière à focaliser le rayonnement sur un point de faible dimensions, dont le diamètre sera de préférence de l'ordre de 200 à 600 microns.

Lorsqu'on procède au découpage de la dentine ou autre matière à l'aide d'une fibre optique en contact avec celle-ci, l'extrémité de la fibre en contact avec la dentine ou cette autre matière est détruite. Il est donc souhaitable d'utiliser une fibre relativement longue que l'on remplace après un certain temps d'utilisation.

On peut effectuer des traitements directement sur la dent ou même à l'intérieur des racines par exemple pour couper la dentine et élargir le canal, avec un rayonnement conduit par une fibre optique et produit par l'appareil selon l'invention. L'énergie est dispensée, même si elle détruit aussi la fibre.

L'unique figure du dessin annexé représente une pièce à main destinée à fournir le rayonnement laser sous une forme convenable pour les interventions précédemment décrites.

Cette pièce à main comprend un carter 1 dont la conformation est prévue, conformément aux règles de l'art, en vue de faciliter la manipulation.

A l'intérieur du logement 1a ménagé dans ce carter, est logée une fibre optique 2 entourée d'un support tubulaire ou gaine 3.

De manière avantageuse, un conduit 4 est également installé ou ménagé à l'intérieur du carter 1, parallèlement ou sensiblement parallèlement à la fibre optique 2.

La pièce à main comprend également une tête de travail 5 qui peut être rapportée, par exemple par vissage, sur l'une des extrémités du carter 1, ou qui peut être formée par une conformation ou par un agencement de ladite extrémité.

A l'intérieur de cette tête de travail, est installé un dispositif optique de focalisation 6 qui, selon l'exemple illustré est constitué par un miroir convergent porté par un support 7 fixé par vissage sur la tête 5.

L'extrémité libre de la fibre optique 2, à la sortie du logement 1a, est tenue par une plaque-support adéquate 10, de manière à diriger le rayonnement lumineux sur le miroir 6.

La tête 5 est agencée pour permettre le positionnement d'un instrument optique d'application interchangeable 8 constitué par une fibre optique souple. La tête 5 est, par exemple, pourvue d'un taraudage 5a permettant la fixation, par vissage, de la fibre optique souple 8 dont l'extrémité interne de fixation 8a peut être filetée ou équipée d'un support fileté 9. Le miroir 6 permet de dévier le rayonnement lumineux sur l'extrémité réceptrice de la fibre 8 ; il exerce, en outre, une action de concentration capable de focaliser le rayonnement à la sortie de la fibre 2, sur un point 0 de la fibre 8, situé de préférence près de la sortie de cette dernière. Cette disposition aide à assurer que la lumière sortant de la fibre 8 puisse être concentrée sur un point suffisamment petit de la surface à traiter.

Lorsqu'elles se trouvent positionnées sur la tête 5, les fibres optiques souples 8 s'étendent hors de ladite tête, sur la plus grande partie de leur longueur. Le diamètre des fibres 8 est, de préférence très réduit, de l'ordre de 250 microns si possible.

L'extrémité de la fibre optique 2 d'acheminement du rayon laser aboutit au dispositif de focalisation 6.

Le conduit 4 destiné à amener le fluide de refroidissement tel que mélange air/eau jusqu'à la tête de travail 5, comporte une extrémité coudée 4a orientée en direction de l'extrémité inférieure de la fibre optique souple 8, de façon à permettre la projection de ce fluide de refroidissement sur l'emplacement d'intervention, à proximité de ladite extrémité. Il est relié, de manière adéquate, à une source 11 de production du mélange eau/air de refroidissement.

La source d'émission à laquelle est reliée la fibre optique 2 comprend une source de lumière monochromatique 12 telle qu'un laser ND YAG produisant un rayonnement sur une longueur d'onde de 1,06 $\mu$m, et raccordée à une source d'énergie 13 de commande fournissant des impulsions suffisantes pour que la source de lumière monochromatique 12 produise des impulsions lumineuses conformes aux paramètres désirés. Une platine 14 capable d'influer sur le rayonnement laser au point de doubler sa fréquence, coulisse sur la source 12 et est solidaire d'une poignée de manoeuvre 15 qui permet de la faire coulisser entre la position représentée sur le dessin où la platine 14 est intercalée sur le trajet de la lumière entre la source 12 et la fibre 2, et une position de retrait où la platine 14 ne coupe plus ladite trajectoire. Grâce à cette disposition simple, la pièce à main dispose de la faculté d'appliquer un rayonnement de 1,06 $\mu$m et ou de 0,532 $\mu$m sur le site d'intervention.

**Revendications**

1. - Appareil utilisant l'effet laser pour la vaporisation ou la fusion de matériaux divers dans un environnement fragile ou délicat, notamment : - pour le traitement de la carie dentaire ; - ou pour la découpe et la sculpture de matériaux divers utilisés en art dentaire ou non tel que métaux, ivoire, émail, os ; - ou pour la fusion, sur la dent, d'un composé minéral à base d'hydroxyapatite et de céramique ; - ou pour l'élargissement des canaux dentaires à l'aide d'une fibre optique conduisant l'énergie laser ; caractérisé en ce qu'il comprend un moyen de production d'un rayonnement laser sur une longueur d'onde permettant une plus forte absorption par l'hydroxyapatite que par l'eau ; un moyen associé audit moyen de production tel que ce dernier produise une succession d'impulsions du rayonnement produit, qui présentent une quantité d'énergie, une durée d'impulsion et une fréquence d'impulsions choisies de manière à couper ou à fondre le matériau dans l'environnement, sans entraîner d'effets secondaires nuisibles sur ce dernier ; et un moyen permettant de concentrer le rayonnement sur le matériau en un point suffisamment petit pour provoquer la découpe ou la fusion de celui-ci.

2. - Appareil selon la revendication 1, caractérisé en ce qu'il comprend un laser spécifique de type ND YAG présentant, en combinaison, les caractéristiques ci-après :
- une longueur d'onde de l'ordre de 1,060 μm ;
- une capacité d'émission d'une énergie pulsée comprise entre 0 et 400 millijoules ;
- des durées de pulsations ou impulsions comprises entre 0,3 et 1,2 millisecondes ;
- un taux de répétition minimal de 50 Hz.

3. - Appareil selon l'une des revendications 1 ou 2, comportant également une pièce à main comprenant un carter (1) de forme allongée, dont l'une des extrémités constitue ou est agencée pour constituer une tête (5) dans laquelle est installé un dispositif optique de focalisation (6) auquel aboutit l'extrémité de la fibre optique (2) logée dans ledit carter (1) et permettant de transporter le rayon laser jusqu'audit dispositif de focalisation (6), caractérisée en ce que cette tête (5) est agencée pour recevoir un instrument d'application interchangeable complétant la pièce à main et constitué par une fibre optique souple (8) dont une partie s'étend hors de ladite tête ; et en ce que la pièce à main est pourvue d'un conduit (4) permettant d'amener un fluide de refroidissement tel qu'un mélange air/eau jusqu'à la tête de travail (5) de ladite pièce à main, ce conduit comportant une extrémité coudée (4a) orientée en direction du site d'intervention, à proximité de l'extrémité libre (8b) de la fibre souple d'application (8) positionnée sur ladite tête de travail.

4. - Procédé utilisant l'effet laser, pour la coupe ou la vaporisation de matériaux divers dans un environnement fragile ou délicat, par exemple : - pour la découpe et la sculpture de matériaux divers utilisés en art dentaire ou non tels que métaux, ivoire, émail, os ; - ou pour la fusion d'un composé minéral à base d'hydroxyapatite et de céramique ; - ou pour l'élargissement des canaux dentaires à l'aide d'une fibre optique conduisant l'énergie laser ; caractérisé en ce qu'on applique sur le matériau à traiter un rayonnement laser dont la longueur d'onde permet une plus forte absorption par l'hydroxyapatite que par l'eau et composé d'impulsions qui présentent une quantité d'énergie, une durée d'impulsion et une fréquence d'impulsions permettant la découpe ou la fusion du matériau sans entraîner d'effets secondaires nuisibles dans la matière environnante.

5. - Procédé selon la revendication 4, caractérisé en ce que l'on irradie le matériau à traiter au moyen d'un rayonnement laser présentant les caractéristiques suivantes :
- une longueur d'onde de l'ordre de 1.060 μm ;
- une énergie pulsée comprise entre 0 et 400 millijoules par impulsion ;
- des impulsions d'une durée comprise entre 0,3 et 1,2 millisecondes ;
- une fréquence de l'ordre de 50 Hz.

6. - Procédé suivant l'une des revendications 4 ou 5 pour l'obturation d'un orifice, caractérisé en ce que l'on remplit l'orifice avec une pâte composée d'acide phosphorique, d'eau, de céramique et d'hydroxyapatique et en ce que l'on irradie cette pâte à l'aide d'un rayonnement laser présentant les caractéristiques suivantes :
- une longueur d'onde de l'ordre de 1,06 μm ;
- une énergie de l'ordre de 20 millijoules par impulsion ;
- des impulsions d'une durée de l'ordre de 0,4 milliseconde ;
- une fréquence de l'ordre de 50 Hz.

7. - Procédé selon la revendication 6, caractérisé en ce que l'on défocalise le rayonnement laser, afin qu'il recouvre au moins une partie substantielle de la surface exposée de la pâte qui obture l'orifice.

8. - Procédé selon l'une des revendications 4 ou 5, pour l'ouverture du foramen au moyen d'une fibre optique, caractérisé en ce que l'on envoie, par l'intermédiaire de cette fibre, une succession d'impulsions d'un rayonnement laser présentant les caractéristiques suivantes :
- une longueur d'onde de l'ordre de 1,06 μm ;
- une énergie de l'ordre de 100 millijoules par impulsion ;
- des impulsions d'une durée de l'ordre de 0,4 milliseconde ;
- une fréquence de l'ordre de 50 Hz.

9. - Procédé pour la découpe et la sculpture de matériaux divers tels que métaux, ivoire, émail, os, selon l'une des revendications 4 ou 5, caractérisé en ce qu'on applique, sur le matériau à découper ou à sculpter un rayonnement laser présentant les caractéristiques suivantes :
- une longueur d'onde de l'ordre de 1,06 μm ;
- une énergie de l'ordre de 400 millijoules par impulsion ;

- des impulsions d'une durée de l'ordre de 0,8 à 1,2 millisecondes ;
- une fréquence de l'ordre de 50 Hz ;

ledit procédé étant encore caractérisé en ce que l'on concentre les impulsions du rayonnement sur un point suffisamment petit du matériau à traiter pour provoquer la découpe de celui-ci et en ce que l'on dirige simultanément, un fluide de refroidissement, par exemple constitué par un mélange air/eau, sur le site d'intervention.

10. - Procédé selon la revendication 9, caractérisé en ce que l'on focalise ou concentre le rayonnement laser sur un point ayant un diamètre de l'ordre de 200 à 600 microns, sur le site d'intervention, et en ce que l'on crée, préalablement, de préférence, une zone sombre au point d'induction.

Fig.1

EP 0 392 951 A2